# EUROPÄISCHE PATENTSCHRIFT

(11) **EP 3 212 825 B1**
(45) Veröffentlichungstag und Bekanntmachung des Hinweises auf die Patenterteilung: **14.11.2018**
(21) Anmeldenummer: 15730379.3
(22) Anmeldetag: 12.05.2015
(51) Int. Cl.: D01F 1/10, A61K 8/02, D01F 2/00, A61Q 1/14, A61Q 19/10, A61Q 17/00, A61K 8/27, A61K 8/73

(54) **FLÜSSIQKEITSQETRÄNKTER VLIESSTOFF, ENTHALTEND ZINKOXID-HALTIGE CELLULOSEFASERN**
LIQUID-IMPREGNATED NONWOVEN FABRIC WHICH CONTAINS ZINC OXIDE-CONTAINING CELLULOSE FIBERS
NON-TISSÉ IMPRÉGNÉ DE LIQUIDE, COMPORTANT DES FIBRES DE CELLULOSE CONTENANT DE L'OXYDE DE ZINC

(30) Priorität: 28.10.2014 AT 7902014
(43) Veröffentlichungstag der Anmeldung: 06.09.2017
(73) Patentinhaber: Lenzing AG, 4860 Lenzing (AT)
(72) Erfinder: HÄUBL, Martin, 4040 Linz (AT)
(74) Vertreter: Hanemann, Otto
(86) Internationale Anmeldenummer: PCT/AT2015/000073
(87) Internationale Veröffentlichungsnummer: WO 2016/065376

(56) Entgegenhaltungen:
- EP-A1- 1 618 240
- EP-B1- 2 334 853
- WO-A1-2009/006206
- CN-B- 101 985 781
- JP-A- 2006 249 615
- JP-A- 2007 191 801
- US-A1- 2011 224 637

## Beschreibung

Die vorliegende Erfindung betrifft einen Vliesstoff, der Cellulosefasern enthält, die zumindest teilweise Zinkoxid-Partikel inkorporiert enthalten und der zusätzlich mit einer Flüssigkeit getränkt ist, sowie ein Verfahren zu seiner Herstellung und seine Verwendung, insbesondere zur Herstellung konservierungsmittelfreier feuchter Wischtücher.

### Stand der Technik

Vor dem Hintergrund sich stark verändernder Lebensumstände und - gewohnheiten, darunter steigende Mobilität, steigender Wohlstand, höhere hygienische Anforderungen und ein zunehmender Mangel an Zeit, ist in den letzten Jahren der Bedarf an feuchten Reinigungstüchern, sogenannten Wet Wipes, die zum Reinigen von harten und weichen Oberflächen eingesetzt werden, stark gestiegen. Es handelt sich dabei um Trägermaterialien, bevorzugt Vliese, die mit einer wirk- bzw. aktivstoffhältigen Tränklösung behandelt, abgepackt und in feuchtem Zustand beispielsweise als Erfrischungs-, Haushalts-, Toilette-, Abschmink- oder Babytücher in den Handel gebracht werden. Die Haupteinsatzgebiete liegen in der Kosmetik (Personal Care, Baby Care) und in der Objektreinigung. Je nach Anwendungsgebiet stehen hinsichtlich der Produktanforderung eine hohe Reinigungsleistung und / oder eine hautpflegende Wirkung im Vordergrund.

Zur Verhinderung der mikrobiellen Kontamination müssen gemäß der Norm Kosmetische Mittel - Mikrobiologie - Bewertung des antimikrobiellen Schutzes eines kosmetischen Produktes (ISO 11930) sämtliche kosmetische Produkte eine ausreichende Konservierung aufweisen. Zu diesem Zweck werden üblicherweise Konservierungsmittel zugesetzt, wodurch zum einen die Haltbarkeit und Lagerfähigkeit des ungeöffneten Produktes sichergestellt, und zum anderen die Verkeimung der Packung nach deren Anbruch verhindert wird. In der Gebrauchsphase stellt der Vorgang der Tuchentnahme durch den Endverbraucher die Haupteintragsquelle für Keime dar. Für die Zwecke der vorliegenden Erfindung sollen unter dem Begriff "Konservierungsstoffe" die Stoffe verstanden werden, die in Annex VI der EU-Kosmetikverordnung (76/768/EEC), Stand 03. September 2014 als solche definiert sind.

Bei Konservierungsmittel handelt es sich um antimikrobiell wirksame Biozide, die das Wachstum von Mikroorganismen wie Bakterien, Pilzen und Hefen unterbinden oder hemmen bzw. diese abtöten. Dies kann sich jedoch auch negativ auf die Hautflora auswirken und sich in einem vermehrten Auftreten von Hautreizungen und -rötungen, Allergien und Ekzemen äußern. Daher ist besonders bei geschädigter oder empfindlicher Haut wie jener von Babys der Zusatz von konservierenden Stoffen zu kosmetischen und medizinischen Produkten kritisch zu sehen. Ungewiss ist in diesem Zusammenhang, ob und in welcher Menge derartige Wirksubstanzen über die Haut aufgenommen werden. Die Datenlage hinsichtlich Toxikologie muss in vielen Fällen als unzureichend eingestuft werden. So legen einige Tierversuche die Vermutung nahe, dass Parabene das Hormonsystem über eine längere Nutzungsdauer beeinflussen können. Phenoxyethanol, ein häufig eingesetztes Konservierungsmittel besitzt möglicherweise eine leberschädigende Wirkung.

Daher ist es notwendig, verträgliche und gleichzeitig wirksame Alternativen zu Konservierungsmitteln zu finden. Zinkoxid wird in der Medizin aufgrund seiner antiseptischen und entzündungshemmenden Wirkung seit langem als Wirkstoff in Produkten zur Hauptpflege und Wundbehandlung eingesetzt. Angeboten werden zinkoxidhältige Salben, Pflaster oder Verbände, die zur oberflächlichen Anwendung bei Hautrötungen, -ausschlägen und - entzündungen (beispielsweise Windeldermatitis) und leichten Verbrennungen geeignet sind. Zink stellt ein für den menschlichen Körper essentielles Spurenelement dar, das wesentlich die Abwehrkräfte und das Funktionieren zahlreicher körpereigener Enzyme beeinflusst. Daher besitzen zinkhältige Produkte eine hohe biologische Verträglichkeit.

Nach dem Stand der Technik werden in der Herstellung von Feuchttüchern sowohl die hautpflegende und heilende Wirkung von Zinkoxid, als auch dessen antimikrobiellen Eigenschaften genutzt, indem unterschiedliche, beispielsweise cellulosische Trägermaterialien in Form von Vliesen mit zinkoxidhältigen Emulsionen oder sonstigen Tränklösungen behandelt werden. Dies ist unter anderem in der US20060171971 (A1) und in der WO9959540 (A1) beschrieben. In den erwähnten Patenten wird auch auf die Option hingewiesen, den flüssigen Formulierungen Konservierungsmittel zuzusetzen. Offensichtlich hat das Zinkoxid in den in diesen Dokumenten beschriebenen Anwendungsformen nur eine pflegende und heilende, aber keine konservierende Wirkung.

Ein wesentlicher Nachteil dieser Produkte besteht darin, dass den Tränkflüssigkeiten mitunter hohe Mengen an Hilfschemikalien, wie Tenside oder Verdickungsmittel zur Einstellung der Viskosität, hinzugefügt werden müssen, um ein unerwünschtes Absetzen der Zinkoxidpartikel während des Herstellprozesses und der Lagerung der Produkte zu vermeiden, welches aufgrund der hohen spezifischen Dichte der ZnO Partikel auftreten würde.

Die Inkorporation von Zinkoxid in Polyolefinmassen zur Herstellung von Nonwovens nach dem Schmelzspinn- bzw. Meltblownverfahren und die Verwendung derartiger antimikrobieller Produkte im Medizin- und Hygienebereich, beispielsweise als feuchte Wischtücher, wird in der JP2006249615 (A) beansprucht. Derartige Nonwovens besitzen aber aufgrund des hydrophoben Charakters der Polyolefine eine geringe Saugkraft und ein niedriges Absorptionsvermögen für wässrige Flüssigkeiten und sind daher nur sehr bedingt als Feuchttücher in Kombination mit wasserhältigen Formulierungen geeignet. Zudem sind diese Vliese im Gegensatz zu Nonwovens aus cellulosischen Fasern nicht biologisch abbaubar.

Auch aufgrund ihrer Hydrophilie, die auf der Eigenschaft der Zellulose beruht, mit Wassermolekülen starke Wasserstoffbrücken zu bilden, und der damit verbundenen hohen Absorptionskraft für wässrige Flüssigkeiten sind cellulosische Fasern Chemiefasern wie Polyester oder Polypropylen in der Herstellung von feuchten Wischtüchern vorzuziehen. Neben Zellstoff werden cellulosische Kunstfasern, sogenannte Celluloseregeneratfasern, wie Viskose- oder Lyocellfasern, eingesetzt. Letztere sind natürlichen Cellulosefasern in Bezug auf Gleichmäßigkeit, Reinheit (z.B. Pestiziderückstände), Weichheit und Saugeigenschaften in weiten Bereichen überlegen. Insbesondere Lyocellfasern zeichnen sich durch eine im Vergleich zu anderen cellulosischen Fasern hohe Trocken- und vor allem Nassfestigkeit aus und ermöglichen die Herstellung weicher Endprodukte mit hohen Festigkeiten.

Gegenstände des täglichen Bedarfs, die zinkoxidhältige Lyocellfasern umfassen und zudem antimikrobielle oder hautfreundliche und heilende Eigenschaften aufweisen, sind beispielsweise aus der DE202010010803 (U1) in Form von sensitiver Wäsche oder der DE202012011814 (U1) in Form eines Fingerlings zur Mund- oder Zahnpflege für Neugeborene und Säuglinge bekannt, wobei letzterer ebenfalls bevorzugt in Form eines Textils ausgeführt ist.

Insgesamt werden also in der Literatur zahlreiche Möglichkeiten genannt, cellulosische, zinkoxidhältige Feuchttücher mit wundheilender, hautpflegender oder antimikrobieller Wirkung herzustellen. Diese Verfahren beschränken sich jedoch durchwegs auf Imprägnierungsverfahren, die dadurch gekennzeichnet sind, dass die Trägermaterialien in Form von Vliesen mit zinkoxidhältigen, flüssigen Formulierungen beladen werden. Diesen Tränkflüssigkeiten werden häufig Konservierungsmittel zur Sicherstellung der Haltbarkeit und weitere Hilfsstoffen wie Tenside, um ein Absetzen der Zinkoxidpartikeln in der Produktion und Lagerung zu verhindern, zugesetzt. Der Einsatz von chemischen Zusatzstoffen und insbesondere von Konservierungsstoffen ist jedoch im Hinblick auf den Verbraucherschutz und die Verbrauchergesundheit unerwünscht.

Vliese mit antimikrobiellen Eigenschaften, hergestellt aus Zinkoxid-haltigen Lyocellspinnmassen nach dem Meltblownverfahren, werden in der WO2009006206 (A1) beschrieben. Die WO2012034679 (A1) beansprucht Spinnvliese, die nach bekannten Spinnvliesverfahren aus mit Metalloxid dotierten cellulosischen Lösungen unter Verwendung von N-Methylmorpholin-N-Oxid als Direktlösungsmittel hergestellt werden. In beiden Patenten wird auf die Verwendung derartiger Vliese im hygienischen Bereich, nicht aber als feuchte Wischtücher im Besonderen eingegangen. Darüber hinaus unterliegen die genannten Vliesstoffe nicht nur Einschränkungen hinsichtlich ihrer Herstellungsverfahren. So beträgt der Hemicellulosegehalt der in der WO2009006206 (A1) beschriebenen Vliese 4 bis 18 %; in der WO2012034679 (A1) ist der Additivgehalt in der Faser auf größer 40 Gew.-% festgelegt. In beiden Fällen sind daher die textilmechanischen Eigenschaften der Fasern stark herabgesetzt. Diese Fasern zeigen unter anderem eine geringe Reißfestigkeit.

Ebenfalls offenbart sind Zinkpigment-haltige Lyocellfasern in EP 2334853 B1. Dort wird eine spezielle Form solcher Fasern beschrieben, in denen das Zink zumindest teilweise als Zinkat vorliegt. Dem Fachmann ist bekannt, dass Zinkat nur in alkalischer Umgebung vorliegen kann. Diese Fasern sollen unter anderem eine antibakterielle Wirkung haben, die insbesondere dadurch erreicht wird, dass im Pigment neben Zinkoxid noch Zinksulfid enthalten ist. Die vordergründige Zielsetzung dieser Faser liegt bei einer Beibehaltung dieser Eigenschaften auch nach mehreren Waschvorgängen. Da die in feuchten Wischtüchern, beispielsweise Babywischtüchern, enthaltenen Lotionen stets einen sauren pH-Wert aufweisen, wird in solchen Anwendungen kein Zinkat auftreten. Der pH Wert der menschlichen Haut ist meist um pH 5,5 wobei die Lotionen in Wischtüchern meist etwas saurer sind um die Haut nicht negativ zu beeinflussen.

WO 2004081267 A1 offenbart im dortigen Beispiel V konkret die Herstellung einer ZnO-haltigen Lyocell-Faser mit einem bestimmten UV-Absorptionsvermögen. Konkrete Verwendungen dieser Faser werden in WO 2004081267 A1 nicht genannt; aus der Beschreibung kann aber geschlossen werden, dass die WO 2004081267 A1 die Herstellung UV-absorbierender Fasern zum Ziel hat.

### Aufgabe

Die Aufgabe bestand gegenüber dem beschriebenen Stand der Technik darin, einen Vliesstoff zur Verfügung zu stellen, der bei Verwendung in feuchten Weiterarbeitungsprodukten keinen zusätzlichen Konservierungsstoff bzw. nur einen deutlich geringeren Gehalt an Konservierungsstoffen als bisher üblich benötigt, insbesondere um bei der Herstellung, Lagerung und Entnahme dieser feuchten Produkte aus der Verpackung eine Verkeimung zu verhindern und den Kontakt der Verbraucher mit solchen Konservierungsstoffen zu minimieren. Dieser Vliesstoff soll aber neben der oben genannten Funktionalität weiterhin optimale Eigenschaften hinsichtlich Nassfestigkeit, Feuchteaufnahme usw. aufweisen.

### Beschreibung der Erfindung

Die Lösung der oben beschriebenen Aufgabe besteht in der Bereitstellung eines Vliesstoffes, der Cellulosefasern enthält, wobei die Cellulosefasern zumindest teilweise Zinkoxid-Partikel inkorporiert enthalten und der Vliesstoff zusätzlich mit einer Flüssigkeit getränkt ist, und wobei die Flüssigkeit einen pH zwischen 4,0 und 5,5 aufweist. Bei den Zinkoxid-haltigen Cellulosefasern handelt es sich erfindungsgemäß um cellulosische Kunstfasern, hergestellt z.B. nach dem Viskose-, Modal- oder Lyocell-Verfahren, die dem Fachmann grundsätzlich bekannt sind. Auch das Einspinnen von festen Stoffen in solche Fasern ist dem Fachmann grundsätzlich bekannt, beispielsweise aus der WO 2011/026159 für Viskosespinnlösungen oder aus der WO 2007/022552 für Lyocellspinnlösungen. Neben weiteren Vorteilen weist der erfindungsgemäße Vliesstoff aufgrund der enzymdeaktivierenden Eigenschaften von ZnO sowie der Adsorption von allergenen Substanzen auch noch antiallergene Eigenschaften auf. Ein überraschender weiterer Vorteil der vorliegenden Erfindung ist, daß diese Eigenschaften hier mit wesentlich geringeren ZnO-Gehalten erreicht werden, als dies nach dem Stand der Technik möglich ist (siehe auch die unten beschriebenen Beispiele und Tabelle 2).

Bevorzugt ist ein Vliesstoff, bei dem die Flüssigkeit einen pH zwischen 4,5 und 5,5 aufweist. Dies wird durch das Tränken des Vliesstoffes mit einer sauren Flüssigkeit mit entsprechendem pH-Wert erreicht. Eine bevorzugte Ausführungsform der vorliegenden Erfindung ist daher ein Vliesstoff, in dem die Flüssigkeit eine organische bzw. anorganische Säure enthält, die ein lösliches Zinksalz bildet. Für die Zwecke dieser Erfindung soll unter einem löslichen Zinksalz ein Zinksalz verstanden werden, welches eine Löslichkeit von mehr als 50 ppm in 18-MOhm-Wasser bei 25°C und einem Luftdruck von 1013 hPa aufweist. Unter "18-MOhm-Wasser" wird allgemein ein über lonentauscher oder sonstige bekannte Verfahren entionisiertes Wasser verstanden, das eine Leitfähigkeit von maximal 18 MOhm aufweist.

Diese Säure sollte in der Kosmetik bereits weit verbreitet sein. Bevorzugt ist diese Säure ausgewählt aus der Gruppe, enthaltend Ameisensäure, Essigsäure, Milchsäure, Zitronensäure, Gluconsäure, Glutaminsäure, Bernsteinsäure, Salzsäure und Schwefelsäure und das lösliche Zinksalz ist dementsprechend bevorzugt ausgewählt aus der Gruppe enthaltend Zinkformiat, Zinkacetat, Zinklactat, Zinkcitrat, Zinkgluconat, Zinkglutamat, Zinksuccinat, Zinkchlorid und Zinksulfat.

Die Zinkoxid-inkorporierten Cellulosefasern im erfindungsgemäßen Vliesstoff enthalten zwischen 0,1 und 10 Gew.-% Zinkoxid, bevorzugt zwischen 0,3 und 4,5 Gew.-% Zinkoxid, insbesondere bevorzugt zwischen 0,3 und 3,5 Gew.-% Zinkoxid, bezogen auf absolut trockene Cellulose. Auch bis zu 17 Gew.-% Zinkoxid sind möglich, aber werden aufgrund wirtschaftlicher Erwägungen nicht häufig verwendet werden.

Der erfindungsgemäße Vliesstoff kann außer den Zinkoxid-inkorporierten Cellulosefasern auch Zinkoxid-freie Cellulosefasern enthalten. Hierfür kommen neben den cellulosischen Kunstfasern, hergestellt z.B. nach dem Viskose-, Modal- oder Lyocell-Verfahren, auch natürliche Cellulosefasern wie Baumwolle, Hanf, etc. in Frage.

Der erfindungsgemäße Vliesstoff kann außer den Zinkoxid-inkorporierten Cellulosefasern auch Synthesefasern enthalten. Hierfür kommen grundsätzlich alle bekannten Synthesefasern in Frage. Besonders geeignet sind Polyethylenterephthalat und Polypropylen.

Im erfindungsgemäßen Vliesstoff sind die Cellulosefasern entweder Stapelfasern oder Endlosfilamentfasern. Zu den Vliessstoffen aus Endlosfilamentfasern können dabei auch die nach dem Meltblown-Verfahren (siehe beispielsweise EP 1093536 B1) hergestellten Vliesstoffe gezählt werden.

Der erfindungsgemäße Vliesstoff weist eine Faserrauhigkeit von 8,5 N oder höher - gemessen mit dem bei den Beispielen beschriebenen Sledgetest - auf. Es wurde gefunden, dass höhere Faserrauhigkeit zu einer besseren Reinigungswirkung führt, unter anderen auch durch Erhöhung der Reinigungsoberfläche des feuchten Wischtuchs.

Gegenstand der vorliegenden Erfindung ist auch ein Verfahren zur Herstellung eines Vliesstoffes mittels herkömmlicher Vliesherstellungsverfahren, in dem zur Vliesbildung Cellulosefasern, die inkorporiertes Zinkoxid enthalten, eingesetzt werden und der Vliesstoff mit einer Flüssigkeit getränkt wird, wobei die Flüssigkeit einen pH zwischen 4,0 und 5,5 aufweist. Als Vliesbildungsverfahren kommen mechanische Vliesbildung, hydrodynamische Vliesbildung, aerodynamische Vliesbildung sowie Meltblowing in Frage; als Vliesverfestigungsverfahren kommen vor allem Wasserstrahlverfestigung, Vernadelung, thermische Verfestigung und chemische Verfestigung in Frage.

Weiterhin ist Gegenstand der vorliegenden Erfindung die Verwendung eines Vliesstoffes, der Zinkoxid-inkorporierte Cellulosefasern enthält und mit einer Flüssigkeit getränkt ist, die einen pH zwischen 4,0 und 5,5 aufweist, zur Herstellung von feuchten Wischtüchern, insbesondere als Babyfeuchttücher, Abschminktücher, Reinigungstücher, etc. Besonders bevorzugt ist eine erfindungsgemäße Verwendung, wobei die feuchten Wischtücher keine zusätzlichen Konservierungsstoffe enthalten. Unter "Konservierungsstoffe" werden hier die Stoffe verstanden, die in Annex VI der EU-Kosmetikverordnung (76/768/EEC), Stand 03. September 2014 aufgelistet sind.

Somit umfasst die vorliegende Erfindung auch feuchte Wischtücher, die den oben beschriebenen, erfindungsgemäßen Vliesstoff enthalten und damit keine solchen zusätzlichen Konservierungsstoffe benötigen.

Eine verbesserte Reinigungskraft tritt auch auf durch Adsorption von anionischem Schmutz (z.B. Fäkalien, organische Säuren usw.) an ZnO an der Oberfläche und im Faserquerschnitt.

Weiterhin ist Gegenstand der vorliegenden Erfindung auch die Verwendung eines Vliesstoffes, der Zinkoxid-inkorporierte Cellulosefasern enthält und mit einer Flüssigkeit getränkt ist, zur Herstellung von feuchten Gesichtsmasken. Solche feuchten Gesichtsmasken können mit Substanzen getränkt sein, die eine hautpflegende Wirkung aufweisen. Das in die Faser inkorporierte ZnO selbst, das für seine hautpflegende Wirkung bekannt ist, trägt ebenfalls zur hautpflegenden Wirkung des Gesamtprodukts bei.

Im Folgenden wird die Erfindung anhand von Beispielen beschrieben. Die Erfindung ist jedoch ausdrücklich nicht auf diese Beispiele beschränkt, sondern umfasst auch alle anderen Ausführungsformen, die auf dem gleichen erfinderischen Konzept beruhen.

### Referenzbeispiele

### Herstellung der feuchten Wischtücher:

Es wurden zunächst gemäß dem im Stand der Technik bekannten Lyocell-Verfahren Lyocell-Fasern hergestellt, in die 0,0, 1,2, 3,0 und 16,7 wt% (bezogen auf atro Faser) ZnO eingesponnen wurden, so dass eine Spinnmasse nach dem Lyocell-Prozess entstand, die 13 wt% Cellulose und die oben genannte ZnO-Menge (bezogen auf Cellulose) enthielt. Diese wurde dann nach dem Lyocell-Prozess versponnen (Einzelfasertiter 2,5 dtex, Schnittlänge 38 mm), mit einer Avivage versehen, welche keinerlei konservierende oder biozide Eigenschaften aufweist und danach getrocknet. Aus den so hergestellten Fasern wurden jeweils Nadelvliese (Flächengewicht 60 g/m², 300 Einstiche/cm²) hergestellt, welche anschließend wasserstrahlverfestigt und danach getrocknet wurden (auf einer Fleissner-Anlage mit folgenden Einstellungen: 35 bar Wasserdruck; 1 Düsenbalken mit 2 Düsenreihen; VE-Waser: pH 7,6; Durchlaufgeschwindigkeit des Vlieses 2 m/min; 100 µm Lochdurchmesser der Wasserstrahldüsen; 118°C Trocknungstemperatur). Die so hergestellten Vliese wurden mit 290 wt% Lotion (bezogen auf das Trockengewicht des Vlieses) getränkt, um entsprechende Baby-Feuchttücher zu erhalten. Die genaue Zusammensetzung der Lotion ist in Tabelle 1 angegeben. Der pH-Wert der Lotion war nach 48 h Kontakt mit den Vliesstoffen auf pH 3,00 eingestellt.

**Tabelle 1:**

| INCI Name | Handelsname | Hersteller | Funktion | wt% |
|---|---|---|---|---|
| Aqua | VE-Wasser | selbst | Lösungsmittel | 96,15 |
| Propylene Glycol | Propylenglycol | Sigma Aldrich | Feuchthaltemittel | 3,00 |
| Polysorbate 20 | Tween 20 | Sigma Aldrich | Lösungsvermittler | 0,60 |
| Capryl/Capramidopropyl Betaine (35 %) | Tego Betain 810 | Evonik | Tensid | 0,25 |
| Acetic acid | Essigsäure pA | Sigma Aldrich | pH-Einstellung | 1,44 |

### Test auf ausreichende Konservierung gemäß ISO 11930:

Je 100 Wischtücher, bestehend aus Fasern mit 0,0, 1,2, bzw. 16,7 wt% ZnO der Größe 15 x 10 cm wurden mit Lotion getränkt (290 wt% Lotion auf Trockengewicht Vlies, Zusammensetzung gemäß Tabelle 1) und daran die Tests auf ausreichende Konservierung durchgeführt. Zu diesem Zweck wurden die Wischtücher mit einem Inocculum beimpft, welches in der Kosmetik übliche Keime in einer definierten Konzentration beinhaltete (S. aureus: 7,40E+05 KBE; P. aeruginosa: 2,80E+05 KBE; E. Coli: 4,40E+05 KBE; C. albican: 6,90E+05 KBE; A. brasiliensis: 1,70E+05 KBE). Nach dem Beimpfen wurden die Wischtücher 28 Tage gelagert. Nach einer Beobachtungszeit von 7, 14 und 28 Tagen wurde jeweils die Keimzahl der Mikroorganismen auf den feuchten Wischtüchern bestimmt (siehe Tabelle 2) um eine Keimreduktion bestimmen zu können.

Die Tests nach ISO 11930 zeigten, dass die Konservierung hinsichtlich der in der Kosmetik üblichen Keime für die Wischtücher mit 1,2 und 16,7 wt% ZnO auf atro Faser erfolgreich war, da eine ausreichende Keimreduktion erreicht wurde. Die höhere ZnO-Menge zeigte eindeutig eine bessere Wirkung (Kriterium A gemäß DIN EN 11930) als die geringere ZnO-Konzentration (Kriterium B gemäß DIN EN 11930).

### Sledgetest gemäß EN 1202 PPS:

Die Weichheit der Fasern wurde mit dem Sledgetest bestimmt, der in EN 1202 PPS beschrieben ist. Die wesentlichen Bedingungen dieses Tests sind: 5 g Faserprobe werden zweifach kardiert, beispielsweise auf einem Uster MTDA-3 Rotorring-Gerät. Die Fasern werden gemäß der EDANA-Vorschrift (ERT 60.2-99) für mindestens 24 h konditioniert und danach mittels einer Schablone geschnitten. Das Material wird anschließend in die Testvorrichtung gegeben und ein Schlitten, der ein Gewicht von 2000 g trägt, wird montiert und auf das Muster gelegt. Der Test wird gestartet und nach 10 sec wird die Kraft gemessen, die notwendig ist, um den Schlitten über das Muster zu ziehen. Je weicher die Faseroberfläche ist, umso weniger Kraft wird benötigt, um den Schlitten vorwärts zu ziehen. Der Text wurde an jedem Material viermal wiederholt. Die Ergebnisse sind in Tabelle 3 dargestellt.

**Tabelle 2:**

| **ZnO-Gehalt der Fasern / wt%** | **Beobachtungszeitraum [Tage]** | **Keime** | | | | | **Ergebnis** |
|---|---|---|---|---|---|---|---|
| | | **S. aureus** | **P. aeruginosa** | **E. coli** | **C. albicans** | **A. brasiliensis** | |
| | | **KBE/g** | **KBE/g** | **KBE/g** | **KBE/g** | **KBE/g** | |
| **0,0** | 7 | 20 | < 10 | < 10 | 9,4x10E3 | - | Erfüllt aufgrund unzureichender Keimreduktion weder Kriterium A noch B. Produkt zeigt optisch dunkle Flecken. |
| | 14 | <10 | < 10 | < 10 | 3,0x10E5 | <10 | |
| | 28 | <10 | <10 | <10 | <10 | <10 | |
| **1,2** | 7 | <10 | < 10 | < 10 | >3,Ox10E5 | - | Erfüllt Kriterium B gemäß DIN EN 11930 |
| | 14 | <10 | < 10 | < 10 | 3, 1x1 0E4 | <10 | |
| | 28 | <10 | < 10 | < 10 | 40 | <10 | |
| **16,7** | 7 | <10 | < 10 | < 10 | 1,5x10E5 | - | Erfüllt Kriterium A gemäß DIN EN 11930 |
| | 14 | <10 | < 10 | < 10 | 1,5x10E5 | 3,3x10E4 | |
| | 28 | <10 | < 10 | < 10 | 2,0x10E4 | 4,8x10E4 | |

**Tabelle 3:**

| Probenbezeichnung | Faser-Faserreibung (Mittelwert) [N] | Standardabweichung [N] |
|---|---|---|
| 3,0 wt% ZnO | 9,23 | 0,25 |
| 1,2 wt% ZnO | 6,86 | 0,14 |

## Patentansprüche

1. Vliesstoff, der Cellulosefasern enthält, wobei die Cellulosefasern zumindest teilweise Zinkoxid-Partikel inkorporiert enthalten und der Vliesstoff zusätzlich mit einer Flüssigkeit getränkt ist, **dadurch gekennzeichnet, dass** die Flüssigkeit einen pH zwischen 4,0 und 5,5 aufweist.

2. Vliesstoff gemäß Anspruch 1, wobei die Flüssigkeit eine organische oder anorganische-Säure enthält, die Zinksalze bildet, welche eine Löslichkeit von mehr als 50 ppm in 18-MOhm-Wasser bei 25°C und einem Luftdruck von 1013 hPa aufweisen.

3. Vliesstoff gemäß Anspruch 1, wobei die Zinkoxid-inkorporierten Cellulosefasern zwischen 0,1 und 10 Gew.-% Zinkoxid, bevorzugt zwischen 0,3 und 4,5 Gew.-% Zinkoxid, insbesondere bevorzugt zwischen 0,3 und 3,5 Gew.-% Zinkoxid, bezogen auf absolut trockene Cellulose, enthalten.

4. Vliesstoff gemäß Anspruch 1, der außer den Zinkoxid-inkorporierten Cellulosefasern auch Zinkoxid-freie Cellulosefasern enthält.

5. Vliesstoff gemäß Anspruch 1, der außer den Zinkoxid-inkorporierten Cellulosefasern auch Synthesefasern enthält.

6. Vliesstoff gemäß Anspruch 1, wobei die Cellulosefasern entweder Stapelfasern oder Endlosfilamentfasern sind

7. Vliesstoff gemäß Anspruch 1, der eine Faser-Faserreibung von 8,5 N oder höher - gemessen mit dem Sledgetest - aufweist.

8. Verfahren zur Herstellung eines Vliesstoffes mittels herkömmlicher Vliesherstellungsverfahren, wobei zur Vliesbildung Cellulosefasern, die inkorporiertes Zinkoxid enthalten, eingesetzt werden und dass der Vliesstoff mit einer Flüssigkeit getränkt wird, **dadurch gekennzeichnet, dass** die Flüssigkeit einen pH zwischen 4,0 und 5,5 aufweist.

9. Verwendung eines Vliesstoffes, der Zinkoxid-inkorporierte Cellulosefasern enthält und mit einer Flüssigkeit mit einem pH zwischen 4,0 und 5,5 getränkt ist, zur Herstellung von feuchten Wischtüchern.

10. Verwendung gemäß Anspruch 9, wobei die feuchten Wischtücher keine zusätzlichen Konservierungsmittel enthalten.

11. Verwendung eines Vliesstoffes, der Zinkoxid-inkorporierte Cellulosefasern enthält und mit einer Flüssigkeit getränkt ist, zur Herstellung feuchter Gesichtsmasken mit hautpflegender Wirkung

## Claims

1. A formed fabric containing cellulose fibers, wherein the cellulose fibers contain incorporated zinc oxide particles at least partially and that the formed fabric is additionally impregnated with a liquid, **characterized in that** the liquid has a pH between 4.0 and 5.5.

2. The formed fabric according to Claim 1, wherein the liquid contains an organic or inorganic acid which forms zinc salts which have a solubility of more than 50 ppm in 18-MOhm water at 25°C and an atmospheric pressure of 1013 hPa.

3. The formed fabric according to Claim 1, wherein the zinc-oxide-incorporated cellulose fibers contain between 0.1 and 10 wt% zinc oxide, preferably between 0.3 and 4.5 wt% zinc oxide, in particular preferably between 0.3 and 3.5 wt% zinc oxide relative to absolutely dry cellulose.

4. The formed fabric according to Claim 1, which also contains zinc-oxide-free cellulose fibers aside from the zinc-oxide-incorporated cellulose fibers.

5. The formed fabric according to Claim 1, which also contains synthetic fibers aside from the zinc-oxide-incorporated cellulose fibers.

6. The formed fabric according to Claim 1, wherein the cellulose fibers are either staple fibers or endless filament fibers.

7. The formed fabric according to Claim 1, which has a fiber-fiber friction of 8.5 N or higher - measured with the sledge test.

8. A method for the manufacturing of a formed fabric by traditional fleece manufacturing methods, wherein cellulose fibers containing incorporated zinc oxide are used for the fleece formation and that the formed fabric is impregnated with a liquid, **characterized in that** the liquid has a pH between 4.0 and 5.5..

9. The use of a formed fabric which contains zinc-oxide-incorporated cellulose fibers and is impregnated with a liquid having a pH between 4.0 and 5.5 for the manufacture of moist wet wipes.

10. The usage according to Claim 9, wherein the moist wet wipes contain no additional preservative agents.

11. The use of a formed fabric which contains zinc-oxide-incorporated cellulose fibers and is impregnated with a liquid for the manufacture of moist face masks with skin-caring action.

## Revendications

1. Étoffe non-tissée contenant des fibres de cellulose, les fibres de cellulose incorporant au moins en partie des particules d'oxyde de zinc et l'étoffe non-tissée étant en outre imprégnée d'un liquide, **caractérisée en ce que** le liquide présente un pH compris entre 4,0 et 5,5.

2. Étoffe non-tissée selon la revendication 1, le liquide contenant un acide organique ou inorganique qui forme des sels de zinc présentant une solubilité supérieure à 50 ppm dans de l'eau de 18 MOhms à 25 °C et avec une pression barométrique de 1013 hPa.

3. Étoffe non-tissée selon la revendication 1, les fibres de cellulose à oxyde de zinc incorporé contenant entre 0,1 et 10 % d'oxyde de zinc en masse, de préférence entre 0,3 et 4,5 % d'oxyde de zinc en masse, ou mieux encore entre 0,3 et 3,5 % d'oxyde de zinc en masse rapporté à la cellulose absolument sèche.

4. Étoffe non-tissée selon la revendication 1 comprenant également, outre les fibres de cellulose à oxyde de zinc incorporé, des fibres de cellulose exemptes d'oxyde de zinc.

5. Étoffe non-tissée selon la revendication 1 comprenant également, outre les fibres de cellulose à oxyde de zinc incorporé, des fibres synthétiques.

6. Étoffe non-tissée selon la revendication 1, les fibres de cellulose étant soit des fibres discontinues, soit des filaments continus.

7. Étoffe non-tissée selon la revendication 1 présentant un frottement fibre/fibre de 8,5 N ou plus - mesuré par essai « sledge test ».

8. Procédé de fabrication d'une étoffe non-tissée au moyen de procédés de fabrication de non-tissés conventionnels, utilisant pour la formation du non-tissé des fibres de cellulose contenant de l'oxyde de zinc incorporé et au cours duquel l'étoffe non-tissée est imprégnée d'un liquide, **caractérisé en ce que** le liquide présente un pH compris entre 4,0 et 5,5.

9. Utilisation d'une étoffe non-tissée contenant des fibres de cellulose à oxyde de zinc incorporé et imprégnée d'un liquide dont le pH est compris entre 4,0 et 5,5, pour la fabrication de lingettes humides.

10. Utilisation selon la revendication 9, les lingettes humides ne contenant aucun conservateur supplémentaire.

11. Utilisation d'une étoffe non-tissée contenant des fibres de cellulose à oxyde de zinc incorporé et imprégnée d'un liquide, pour la fabrication de masques humides pour le visage ayant un effet bénéfique pour la peau.
